# EUROPEAN PATENT SPECIFICATION

(11) **EP 4 467 976 B1**
(45) Date of publication and mention of the grant of the patent: **24.06.2026**
(21) Application number: 24176873.8
(22) Date of filing: 20.05.2024
(51) Int. Cl.: G01N 33/00, G01D 11/24

(54) **AN ELECTROMAGNETIC SHIELD FOR A GAS DETECTOR**
ELEKTROMAGNETISCHE ABSCHIRMUNG FÜR EINEN GASDETEKTOR
BLINDAGE ÉLECTROMAGNÉTIQUE POUR DÉTECTEUR DE GAZ

(30) Priority: 22.05.2023 US 202363503563 P
(43) Date of publication of application: 27.11.2024
(73) Proprietor: Detector Electronics Buyer US, LLC, Bloomington, MN 55438 (US)
(72) Inventor: WARNER, Tanner, Minneapolis, MN 55438 (US); EDWARDS, Glen, Minneapolis, MN 55438 (US)
(74) Representative: Dehns

(56) References cited:
- CN-U- 212 904 644
- US-A- 4 098 653
- US-A1- 2008 016 948
- US-A1- 2020 095 118

## Description

The disclosure generally relates to gas detectors. More particularly, the disclosure relates to an electromagnetic shield for a gas detector, and a gas detector.

Gas detectors are widely used to detect the presence of dangerous or toxic gases such as carbon monoxide, hydrogen sulfide, and others, in industrial, commercial, and residential settings. In heating ventilation and air conditioning (HVAC) applications, gas detectors may be employed to detect refrigerant leaks. To protect the sensors of gas detectors from environmental conditions, physical damage, or other factors that may affect their performance, several protective shields have been developed over the years.

**However,** despite their utility, existing protective shields are not without disadvantages. One major issue is that installation or removal of existing protective shields can be difficult. This can increase the time and the cost of implementing gas detectors for safety purposes. As such, existing protective shields can be expensive and cumbersome to install. Therefore, ease of installation or removal of protective shields is desirable to allow regular maintenance of the gas detectors thereby ensuring optimal functioning.

Weather resistance and susceptibility to electromagnetic interference is another issue with existing protective shields. Some protective shields may not be weather resistant, making them susceptible to damage from extreme temperatures, rain, wind, or other environmental conditions. Similarly, the existing protective shields may not be equipped to protect the sensors from electromagnetic interference. This may cause errors in the functioning of the sensors thereby compromising the reliability of the gas detectors over time.

US 2020/0095118 A1 discloses a device for suppressing stray radiation including a MEMS sensor module and a conductive cage structure enclosing the MEMS sensor module. CN 212904644 U discloses an anti-electromagnetic interference gas sensor packaging structure having an air escape cap made of shielding materials.

This summary is provided to introduce a selection of concepts in a simplified format that are further described in the detailed description of the disclosure. This summary is not intended to identify key or essential inventive concepts of the disclosure, nor is it intended for determining the scope of the disclosure.

According to a first aspect of the invention there is provided an electromagnetic shielding member for a gas detector as recited in claim 1. The electromagnetic shielding member includes a hollow body adapted to enclose at least a portion of a sensor. The hollow body includes a connecting portion and at least one airflow channel. The connecting portion is adapted: to electrically connect the hollow body to an enclosure of the gas detector, and to removably fasten the hollow body to an enclosure of the gas detector. The at least one airflow channel is defined in the hollow body and in fluid communication with the sensor such that the at least one airflow channel is adapted to block an electromagnetic signal based on a set of geometrical parameters associated with the at least one airflow channel.

Optionally, the set of geometrical parameters includes at least one of a shape, an internal diameter, an orientation, and a length of the at least one airflow channel.

Optionally, the internal diameter of the at least one airflow channel is less than 5mm.

Optionally, the at least one airflow channel is oriented to prevent at least one of ambient particulate matter and water particles from directly contacting the sensor.

Optionally, the at least one airflow channel is oriented at an inclination greater than 45 degrees relative to a longitudinal axis (X-X') of the hollow body.

Optionally, the connecting portion is defined on an outer surface of a side wall of the hollow body.

Optionally, the side wall includes at least one baffle portion extending downward from the side wall in a direction parallel to the longitudinal axis (X-X') of the hollow body.

Optionally, the hollow body includes at least one baffle portion removably fastened to the side wall and extending downward in a direction parallel to the longitudinal axis (X-X') of the hollow body.

Optionally, the at least one baffle portion is spaced apart from the hollow body to permit entry of ambient air into the at least one airflow channel.

Optionally, the at least one baffle portion and the at least one airflow channel are aligned to permit entry of ambient air into the at least one airflow channel.

According to a second aspect of the invention, there is provided a gas detector as recited in claim 11. The gas detector comprises an enclosure adapted to enclose at least a portion of a sensor and control circuitry electrically connected to the sensor; and an electromagnetic shielding member as described herein with reference to the first aspect of the invention, adapted to be removably fastened to the enclosure. The gas detector may comprise any of the feature of the electromagnetic shielding member as described herein, and/or any of the features of a gas detector as described herein.

According to another aspect of the invention, there is provided a gas detector, including an enclosure and an electromagnetic shielding member. The enclosure is adapted to enclose at least a portion of a sensor and control circuitry electrically connected to the sensor. The electromagnetic shielding member is adapted to be removably fastened to the enclosure. The electromagnetic shielding member includes a hollow body and at least one airflow channel defined in the hollow body. The hollow body is adapted to enclose the sensor. Moreover, the hollow body includes a connecting portion adapted to: electrically connect the hollow body to the enclosure, and to removably fasten the hollow body to an enclosure of the gas detector. The at least one airflow channel is in fluid communication with the sensor, such that the at least one airflow channel is adapted to block an electromagnetic signal based on a set of geometrical parameters associated with the at least one airflow channel.

Optionally, the set of geometrical parameters comprise at least one of a shape, an internal diameter, an orientation, and a length of the at least one airflow channel.

Optionally, the internal diameter of the at least one airflow channel is less than 5mm.

Optionally, the at least one airflow channel is oriented to prevent at least one of ambient particulate matter and water particles from directly contacting the sensor.

Optionally, the at least one airflow channel is oriented at an inclination greater than 45 degrees relative to a longitudinal axis (X-X') of the hollow body.

Optionally, the connecting portion is defined on an outer surface of a side wall of the hollow body.

Optionally, the side wall includes at least one baffle portion extending downward from the side wall in a direction parallel to the longitudinal axis (X-X') of the hollow body.

Optionally, the at least one baffle portion is adapted to removably fasten a secondary cover element to the side wall.

Optionally, the at least one baffle portion is spaced apart from the hollow body to permit entry of ambient air into the at least one airflow channel.

Optionally, the at least one baffle portion and the at least one airflow channel are aligned to permit entry of ambient air into the at least one airflow channel.

**The** gas detector of this aspect may be a gas detector as recited herein with reference to the second aspect of the invention, and may comprise any of the features of a gas detector as recited herein, and/or of the feature of an electromagnetic shielding member as recited herein. The gas detector of the second aspect of the invention may comprise any of the features of a gas detector recited herein.

**To** further clarify the advantages and features of the methods, systems, and apparatuses, a more particular description of the methods, systems, and apparatuses will be rendered by reference to specific embodiments thereof, which is illustrated in the appended drawing. It is appreciated that these drawings depict only typical embodiments of the disclosure and are therefore not to be considered limiting its scope. The disclosure will be described and explained with additional specificity and detail with the accompanying drawings.

These and other features, aspects, and advantages of the disclosure will become better understood when the following detailed description is read with reference to the accompanying drawings which are provided by way of example only and in which like characters represent like parts throughout the drawings, wherein:
**FIG. 1A** exemplarily illustrates an isometric view of a gas detector;
**FIG. 1B** exemplarily illustrates a cross-sectional view of the gas detector;
**FIG. 1C** exemplarily illustrates a cross-sectional view of an electromagnetic shielding member of the gas detector;
**FIG. 2A** exemplarily illustrates an isometric view of the gas detector;
**FIG. 2B** exemplarily illustrates a cross-sectional view of the gas detector;
**FIG. 3A** exemplarily illustrates an isometric view of the gas detector;
**FIG. 3B** exemplarily illustrates a cross-sectional view of the gas detector; and
**FIG. 3C** exemplarily illustrates a cut-away view of the electromagnetic shielding member of the gas detector.

Further, skilled artisans will appreciate that elements in the drawings are illustrated for simplicity and may not have necessarily been drawn to scale. For example, the flow charts illustrate the method in terms of the most prominent steps involved to help to improve understanding of aspects of the disclosure. Furthermore, in terms of the construction of the device, one or more components of the device may have been represented in the drawings by conventional symbols, and the drawings may show only those specific details that are pertinent to understanding the embodiments of the disclosure so as not to obscure the drawings with details that will be readily apparent to those of ordinary skill in the art having the benefit of the description herein.

For the purpose of promoting an understanding of the principles of the disclosure, reference will now be made to the various embodiments and specific language will be used to describe the same. It will nevertheless be understood that no limitation of the scope of the disclosure is thereby intended, such alterations and further modifications in the illustrated system, and such further applications of the principles of the disclosure as illustrated therein being contemplated as would normally occur to one skilled in the art to which the disclosure relates.

It will be understood by those skilled in the art that the foregoing general description and the following detailed description are explanatory of the disclosure and are not intended to be restrictive thereof.

Reference throughout this specification to "an aspect", "another aspect" or similar language means that a particular feature, structure, or characteristic described in connection with the embodiment is included in at least one embodiment of the disclosure. Thus, appearances of the phrase "in an embodiment", "in another embodiment", "some embodiments", "one or more embodiments" and similar language throughout this specification may, but do not necessarily, all refer to the same embodiment.

The terms "comprises", "comprising", or any other variations thereof, are intended to cover a non-exclusive inclusion, such that a process or method that comprises a list of steps does not include only those steps but may include other steps not expressly listed or inherent to such process or method. Similarly, one or more devices or sub-systems or elements or structures or components proceeded by "comprises... a" does not, without more constraints, preclude the existence of other devices or other sub-systems or other elements or other structures or other components or additional devices or additional sub-systems or additional elements or additional structures or additional components.

Embodiments will be described below in detail with reference to the accompanying drawings.

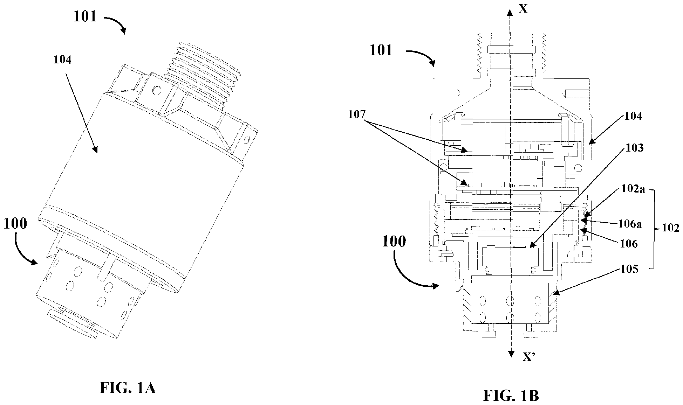

**FIG. 1A** exemplarily illustrates an isometric view of a gas detector 101 according to one or more embodiments of the disclosure. **FIG. 1B** exemplarily illustrates a cross-sectional view of the gas detector 101 according to one or more embodiments of the disclosure. The gas detector 101 includes an enclosure 104 adapted to accommodate at least a portion of a sensor 103 and control circuitry 107 electrically connected to the sensor 103. In an embodiment, the sensor 103 may include, but is not limited to, combustible gas sensors, photoionization sensors, infrared point sensors, ultrasonic sensors, electrochemical gas sensors, and metal-oxide-semiconductor (MOS) sensors 103. The gas detector 101 may also include additional thermal image processing sensors 103. Typically, the sensor 103 may be adapted to detect a presence of a hazardous gas. Alternatively, the sensor 103 may be calibrated to generate a trigger signal if a quantity of the hazardous gas exceeds a threshold level. In an embodiment, the hazardous gas may include, but is not limited to, an oxide of carbon, an oxide of sulphur, an oxide of nitrogen, a polycyclic aromatic hydrocarbon, and an aldehyde.

As shown in **FIGS. 1A-1B****,** the gas detector 101 includes an electromagnetic shielding member 100 adapted to be removably fastened to the enclosure 104. When the enclosure 104 and the electromagnetic shielding member 100 are assembled, the enclosure 104 and the electromagnetic shielding member 100 collectively accommodates the sensor 103, the control circuitry 107, and other sub-components necessary for operation of the gas detector 101. In an embodiment, the gas detector 101 is mounted on a surface, for example a ceiling in a building. Therefore, the enclosure 104 is typically closer to the ceiling than the electromagnetic shielding member 100. As such, when the gas detector 101 is mounted on the ceiling of the building, the electromagnetic shielding member 100 faces downward toward a floor of the building. Alternatively, the gas detector 101 may be mounted on a wall of the building such that the enclosure 104 is closer to the wall than the electromagnetic shielding member 100. In such embodiments, the electromagnetic shielding member 100 is may face outward toward an interior space of a room or space to be monitored.

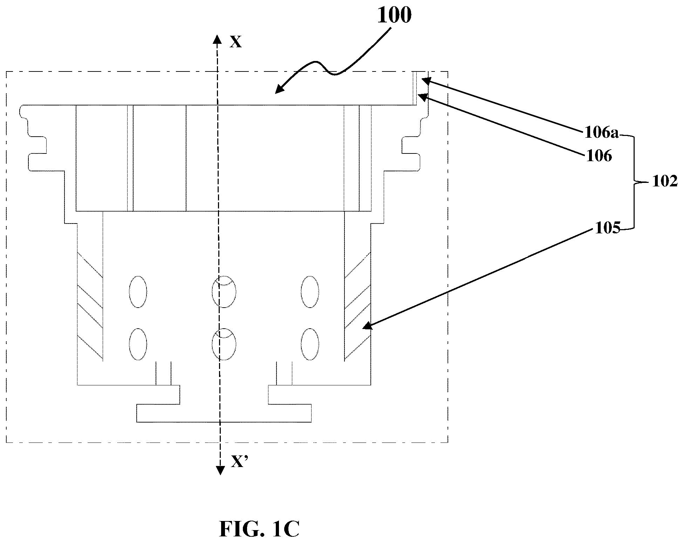

**FIG. 1C** exemplarily illustrates a cross-sectional view of the electromagnetic shielding member 100 according to one or more embodiments of the disclosure. Referring to **FIGS. 1B-1C****,** the electromagnetic shielding member 100 includes a hollow body 102 adapted to enclose the sensor 103. The hollow body 102 includes a connecting portion 102a and at least one airflow channel 105. The connecting portion 102a is adapted to electrically connect the hollow body 102 to the enclosure 104 of the gas detector 101. The connecting portion 102a of the electromagnetic shielding member 100 is electrically connected via a metal surface to metal surface contact. The connecting portion 102a is adapted to removably fasten the hollow body 102 to the enclosure 104 of the gas detector 101. The connecting portion 102a may be defined on an outer surface 106a of a side wall 106 of the hollow body 102. The connecting portion 102a may be defined as external screw threads on the outer surface 106a of the side wall 106 of the hollow body 102. The external screw threads are adapted to threadably engage with screw threads defined on an internal surface of the enclosure 104.

Referring to **FIGS. 1B-1C****,** the at least one airflow channel 105 is defined in the hollow body 102 such that the at least one airflow channel 105 is in fluid communication with the sensor 103. The at least one airflow channel 105 is adapted to block an electromagnetic signal based on a set of geometrical parameters associated with the at least one airflow channel 105. In an embodiment, the set of geometrical parameters includes a shape, an internal diameter, an orientation, or a length of the at least one airflow channel 105 is selected based on one or more parameters of the electromagnetic signal to be blocked. The at least one airflow channel 105 allows ingress of ambient gases into the enclosure 104 such that the sensor 103 detects the ambient gases entering the enclosure 104. In an embodiment, the internal diameter of the at least one airflow channel 105 is less than 5mm. This internal diameter of the at least one airflow channel 105 blocks or attenuates the electromagnetic signals from entering the enclosure 104. By varying the diameter of the at least one airflow channel 105, the electromagnetic signals having different wavelengths or frequencies may be blocked or attenuated. For example, the at least one airflow channel 105 having an internal diameter of 5mm attenuates a 6GHz frequency. Moreover, the at least one airflow channel 105 is oriented to prevent at least one of ambient particulate matter and water particles from directly contacting or striking the sensor 103. In an embodiment, the at least one airflow channel 105 is oriented at an inclination greater than 45 degrees relative to a longitudinal axis (X-X') of the hollow body 102.

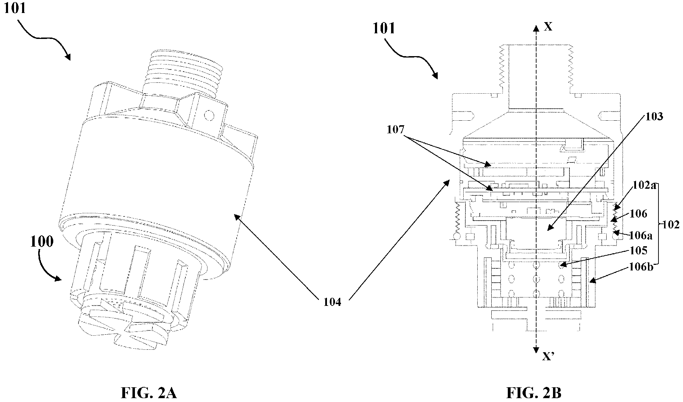

**FIG. 2A** exemplarily illustrates an isometric view of the gas detector 101 according to one or more embodiments of the disclosure. **FIG. 2B** exemplarily illustrates a cross-sectional view of the gas detector 101 according to one or more embodiments of the disclosure. The gas detector 101 includes the enclosure 104 adapted to enclose the sensor 103 and the control circuitry 107 electrically connected to the sensor 103. Moreover, the gas detector 101 includes the electromagnetic shielding member 100 adapted to be removably fastened to the enclosure 104. The electromagnetic shielding member 100 includes the hollow body 102 having the connecting portion 102a and the at least one airflow channel 105. For the sake of brevity, components that have already been described in the detailed descriptions of **FIGS. 1A-1C** have not been described again in detail.

Referring to **FIG. 2B****,** the hollow body 102 of the electromagnetic shielding member 100 includes the side wall 106 having at least one baffle portion 106b extending downward from the side wall 106 in a direction parallel to the longitudinal axis (X-X') of the hollow body 102. In an embodiment, the hollow body 102 is a hollow cylindrical shaped body adapted to accommodate at least a portion of the sensor 103. As shown in **FIG. 2B****,** the at least one baffle portion 106b is merely an extension of the side wall 106 in the embodiment. The at least one baffle portion 106b is therefore concentric or coaxial to the hollow body 102. Moreover, the at least one baffle portion 106b is spaced apart from the hollow body 102 to permit entry of ambient air into the at least one airflow channel 105. In an embodiment, the at least one baffle portion 106b and the at least one airflow channel 105 may be aligned to permit entry of ambient air into the at least one airflow channel 105. Moreover, the alignment of the at least one baffle portion 106b and the at least one airflow channel 105 prevents the ambient particulate matter and/or water particles from directly contacting or striking the sensor 103.

In some embodiments, a plurality of baffle portions 106b may be spaced apart from the hollow body 102 and positioned equidistant relative to each other. In such an embodiment, the plurality of baffle portions 106b may be positioned along the periphery of the hollow body 102. Each of the plurality of baffle portions 106b may be aligned with a corresponding airflow channel 105 to cover the corresponding airflow channel 105. This alignment of each of the plurality of baffle portions 106b with the corresponding airflow channel permits entry of ambient air into the corresponding at least one airflow channel 105. Moreover, the alignment of the at least one baffle portion 106b and the at least one airflow channel 105 prevents the ambient particulate matter and/or water particles from directly contacting or striking the sensor 103.

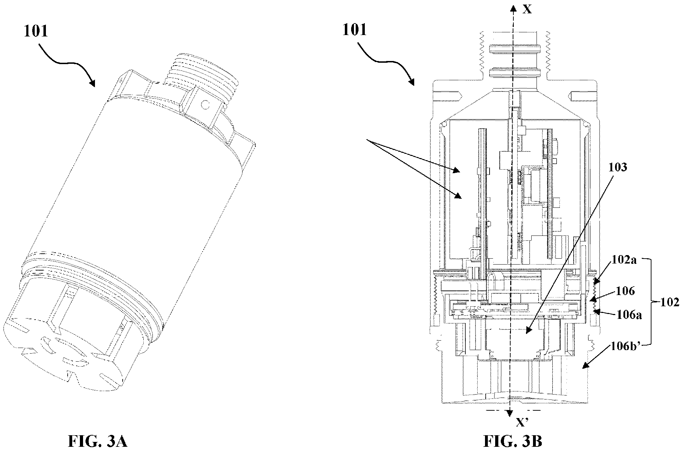

**FIG. 3A** exemplarily illustrates an isometric view of the gas detector 101 according to one or more embodiments of the disclosure. **FIG. 3B** exemplarily illustrates a cross-sectional view of the gas detector 101 according to one or more embodiments of the disclosure. The gas detector 101 includes the enclosure 104 adapted to enclose the sensor 103 and the control circuitry 107 electrically connected to the sensor 103. Moreover, the gas detector 101 includes the electromagnetic shielding member 100 adapted to be removably fastened to the enclosure 104. The electromagnetic shielding member 100 includes the hollow body 102 having the connecting portion 102a. For the sake of brevity, components that have already been described in the detailed descriptions of **FIGS. 1A-1C** have not been described again in detail.

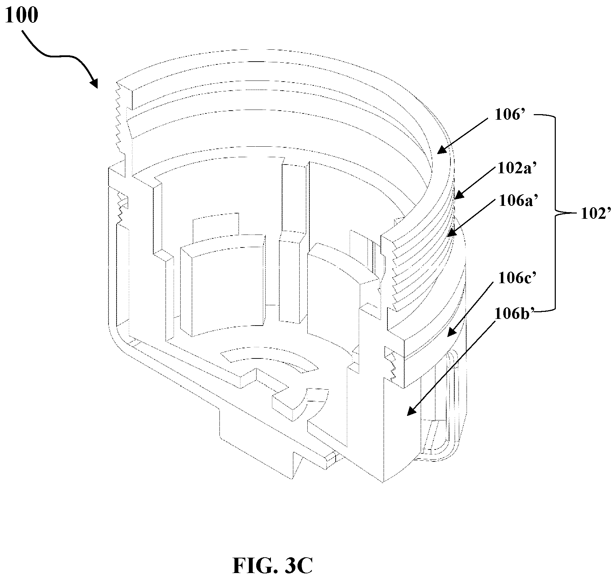

**FIG. 3C** exemplarily illustrates a cut away view of the electromagnetic shielding member 100 according to one or more embodiments of the disclosure. As shown in **FIGS. 3B** and **3C****,** in an embodiment, the at least one baffle portion 106b' may have external or internal threads for removably fastening a secondary cover element 106c' to the side wall 106' of the hollow body 102'. The provision of the secondary cover element 106c' may further prevent the ambient particulate matter or water particles from directly striking or contacting the sensor 103. Moreover, the at least one baffle portion 106b' extends downward in a direction parallel to the longitudinal axis (X-X') of the hollow body 102'. The at least one baffle portion 106b' is concentric or coaxial to the hollow body 102' and spaced apart from the hollow body 102' to permit entry of ambient air into the at least one airflow channel 105' (not shown). In an embodiment, the at least one baffle portion 106b' may not be rotatable relative to the hollow body 102'. The at least one baffle portion 106b' and the at least one airflow channel 105' (not shown) may be aligned to permit entry of ambient air into the at least one airflow channel 105' (not shown). In some embodiments, a plurality of baffle portions 106b', may be spaced apart from the hollow body 102' and positioned equidistant relative to each other.

The electromagnetic shielding member 100, disclosed herein, is made from a metallic material or a conductive plastic material. Alternatively, the electromagnetic shielding member 100 is made of a metallic material such as stainless steel or a non-metallic material, which has a certain amount of electrical conductivity. The amount of electrical conductivity would depend on the one or more parameters, for example, a wavelength, a frequency, etc., of the electromagnetic signal desired to be blocked. In an embodiment, the electromagnetic shielding member 100 may be manufactured from conventional manufacturing techniques (injection moulding and the like) or 3D printing techniques. The electromagnetic shielding member 100 is threaded onto the enclosure 104 of the gas detector 101. Advantageously, this feature of the electromagnetic shielding member 100 allows the electromagnetic shielding member 100 to be easily installed, replaced, or reused after repair. Moreover, this design also ensures lesser number of components for shielding from electromagnetic interference and water particles and ambient particulate matter. Alternatively, the electromagnetic shielding member 100 may not be threaded. Instead, the electromagnetic shielding member 100 may be electrically connected having the at least one airflow channel 105 with a diameter less than 5mm to block electromagnetic signals. Advantageously, this diameter blocks or attenuates the electromagnetic signals from entering the enclosure 104. By varying the diameter, electromagnetic signals having different wavelengths may be blocked/attenuated.

In one or more embodiments, the electromagnetic shielding member 100 is also designed to protect a sensing interface of the sensor 103 from getting directly hit with particles of water. In an embodiment, the at least one baffle portion 106b is positioned in front of the at least one airflow channel 105. The at least one baffle portion 106b may not be rotatable relative to the at least one airflow channel 105 and therefore prevent particles of water from directly striking the sensing interface of the sensor 103. In another embodiment, the at least one airflow channel 105 is oriented at the inclination greater than 45 degree relative to the longitudinal axis (X-X') of the hollow body 102. This arrangement further reduces the chances of the particles of water from directly striking the sensing interface of the sensor 103. Advantageously, this design ensures shielding of the enclosure 104 from electromagnetic interference in addition to water particles and ambient particulate matter. Therefore, the electromagnetic shielding member 100 for gas detectors is weather resistant, easy to install, less susceptible to electromagnetic interference, while remaining cost-effective to implement. As such, the electromagnetic shielding member 100 provides improved protection for the sensor 103 while maintaining the sensitivity and accuracy of the sensor 103.

As used herein, the term "control circuitry 107" may be construed to encompass one or a combination of microprocessors, suitable logic, circuits, printed circuit boards (PCB), audio interfaces, visual interfaces, haptic interfaces, or the like. The control circuitry 107 may include, but is not limited to, a microcontroller, a Reduced Instruction Set Computing (RISC) processor, an Application-Specific Integrated Circuit (ASIC) processor, a Complex Instruction Set Computing (CISC) processor, a central processing unit (CPU), a graphics processing unit (GPU), a state machine, and/or other processing units or circuits.

The control circuitry 107 may also include suitable logic, circuits, interfaces, and/or code that may be configured to execute a set of instructions stored in a memory unit. In an exemplary implementation of the memory unit according to the disclosure, the memory unit may include, but are not limited to, Electrically Erasable Programmable Read-only Memory (EEPROM), Random Access Memory (RAM), Read Only Memory (ROM), Hard Disk Drive (HDD), Flash memory, Solid-State Drive (SSD), and/or CPU cache memory.

In an exemplary embodiment, the control circuitry 107 may receive power from a suitably coupled power source (not shown). For example, a battery or a power source may be electrically coupled to supply electrical power to the control circuitry 107. In an embodiment, the power source may be, for example, a battery, such as a rechargeable battery or a non-rechargeable battery. Examples of suitable batteries include, for example, a lithium battery (such as a lithium-ion battery), a nickel battery (such as a nickel-cadmium battery), and an alkaline battery.

In case one or more threshold levels are breached, the control circuitry 107 may be configured to actuate a notification unit (not shown) to generate a notification. In an embodiment, the notification is, for example, an audio notification, a visual notification, an audio-visual notification, and a haptic notification. In an embodiment, the notification unit may include a sound generation mechanism connected to the control circuitry 107. The notification unit may be actuated by the control circuitry 107 to generate a noise in response to detection of the threshold level of the hazardous gas being breached. The audio notification may include a loud warning siren or alarm which may be generated for a continuous or periodic interval of time.

The control circuitry 107 may also include a communication unit adapted to communicate with other gas detectors of a fire detection system or a hazard detection system. In an embodiment, the communication unit also transmits data to and receives data from other gas detectors 101 via a communication network. The communication unit may be configured of, for example, a telematic transceiver (DCM), a mayday battery, a GPS, a data communication module ASSY, a telephone microphone ASSY, and a telephone antenna ASSY. The communication network may include, but is not limited to, a Wide Area Network (WAN), a cellular network, such as a 3G, 4G, or 5G network, an Internet-based mobile ad hoc networks (IMANET), etc. The communication network may also include wired media such as a wired network or direct-wired connection, and wireless media such as acoustic, radio frequency (RF), microwave, infrared (IR) and other wireless media.

In an embodiment, the communication unit also transmits data to and receives data from a computing device via a communication network. In an embodiment, the computing device may be implemented as a portion of a small-form factor portable (or mobile) electronic device such as a cell phone, a personal data assistant (PDA), a personal media player device, a wireless web-watch device, a personal headset device, an application specific device, or a hybrid device that include any of the above functions. In an embodiment, the control circuitry is further configured to generate a secondary notification on the computing device based on the control circuitry 107 determining presence of the hazardous gas.

In some exemplary implementations according to the disclosure, the computing device may be adapted to generate a visual notification in addition to the audio notification via a display interface of the computing device. The display may include suitable logic, circuitry, interfaces, and/or code that may be configured to render various types of information and/or entertainment content via a user interface. In an embodiment, the display may be a flashing visual indicator, such as a Light Emitting Diode (LED), indicator lights, or the like. The user interface may be a customized Graphic User Interface (GUI) configured to display information related to the fire detection system or the hazard detection system such as the threshold level set by the operator, location of the gas detector 101, etc. The display may include, but is not limited to, a projection-based display, an electro-chromic display, a flexible display, and holographic display. In other embodiments, the display may be a touchscreen display, a tactile electronic display, and/or a touchable hologram. As such, the display may be configured to receive inputs from the operator for setting or modifying the threshold level of the hazardous gas, etc.

In some embodiments, the gas detector 101 may be hardwired to the power source (not shown) located within the building or area where the gas detector 101 is mounted, remote from the gas detector 101. In such embodiments, the control circuitry 107 may be directly or indirectly connected to the power source. In an embodiment, the gas detector 101 may include a compartment for receiving one or more batteries sufficient to provide the power necessary to operate the gas detector 101 for an extended period. In an embodiment, the power provided by the batteries may be the sole source of power used to operate the gas detector 101. However, in other embodiments, the battery power may be supplemental to the remote power source, for example in the event of a failure or loss of power at the power source.

As would be apparent to a person in the art, various working modifications may be made to the method in order to implement the inventive concept as taught herein.

Moreover, the actions of any flow diagram need not be implemented in the order shown; nor do all the acts necessarily need to be performed. Also, those acts that are not dependent on other acts may be performed in parallel with the other acts.

Benefits, other advantages, and solutions to problems have been described above with regard to specific embodiments. However, the benefits, advantages, solutions to problems, and any component(s) that may cause any benefit, advantage, or solution to occur or become more pronounced are not to be construed as a critical, or essential feature or component of any or all the claims.

While specific language has been used to describe the subject matter, any limitations arising on account thereto, are not intended. As would be apparent to a person in the art, various working modifications may be made to the method in order to implement the inventive concept as taught herein. The drawings and the foregoing description give examples of embodiments. Those skilled in the art will appreciate that one or more of the described elements may well be combined into a single functional element. Alternatively, certain elements may be split into multiple functional elements. Elements from one embodiment may be added to another embodiment.

## Claims

1. An electromagnetic shielding member (100) for a gas detector (101), the electromagnetic shielding member (100) comprising:
a hollow body (102) adapted to at least partially enclose a sensor (103), the hollow body comprising:
at least one airflow channel (105) defined in the hollow body (102), the at least one airflow channel in fluid communication with the sensor (103), wherein the at least one airflow channel (105) is adapted to block an electromagnetic signal based on a set of geometrical parameters associated with the at least one airflow channel (105); and **characterized in that**
the hollow body (102) comprises a connecting portion (102a) adapted to electrically connect the hollow body (102) to an enclosure (104) of the gas detector (101) and to removably fasten the hollow body (102) to an enclosure (104) of the gas detector (101).

2. The electromagnetic shielding member (100) of claim 1, wherein the set of geometrical parameters comprises at least one of a shape, an internal diameter, an orientation, and a length of the at least one airflow channel.

3. The electromagnetic shielding member (100) of claim 1 or 2, wherein the internal diameter of the at least one airflow channel (105) is less than 5mm.

4. The electromagnetic shielding member (100) of claim 1, 2 or 3, wherein the at least one airflow channel (105) is oriented to prevent at least one of ambient particulate matter and water particles from directly contacting the sensor (103).

5. The electromagnetic shielding member (100) of any preceding claim, wherein the at least one airflow channel (105) is oriented at an inclination greater than 45 degrees relative to a longitudinal axis (X-X') of the hollow body (102).

6. The electromagnetic shielding member (100) of any preceding claim, wherein the connecting portion (102a) is defined on an outer surface (106a) of a side wall (106) of the hollow body (102).

7. The electromagnetic shielding member (100) of claim 6, wherein the side wall (106) comprises at least one baffle portion (106b) extending downward from the side wall (106) in a direction parallel to the longitudinal axis (X-X') of the hollow body (102).

8. The electromagnetic shielding member (100) of any preceding claim, wherein the hollow body (102) comprises at least one baffle portion (106b) removably fastened to the side wall (106) and extending downward in a direction parallel to the longitudinal axis (X-X') of the hollow body (102).

9. The electromagnetic shielding member (100) of any preceding claim, wherein the at least one baffle portion (106b) is spaced apart from the hollow body (102) to permit entry of ambient air into the at least one airflow channel (105).

10. The electromagnetic shielding member (100) of claim 9, wherein the at least one baffle portion (106b) and the at least one airflow channel (105) are aligned to permit entry of ambient air into the at least one airflow channel (105).

11. A gas detector (101) comprising:
an enclosure (104) adapted to enclose at least a portion of a sensor (103) and control circuitry (107) electrically connected to the sensor (103); and
the electromagnetic shielding member (100) of any preceding claim adapted to be removably fastened to the enclosure (104).

12. The gas detector (101) of claim 11 when dependent upon claim 7, wherein the at least one baffle portion (106b') is adapted to removably fasten a secondary cover element (106c') to the side wall (106').

## Patentansprüche

1. Elektromagnetisches Abschirmelement (100) für einen Gasdetektor (101), das elektromagnetische Abschirmelement (100) umfassend:
einen Hohlkörper (102), der angepasst ist, um einen Sensor (103) mindestens teilweise zu umschließen, der Hohlkörper umfassend:
mindestens einen Luftströmungskanal (105), der in dem Hohlkörper (102) definiert ist, wobei der mindestens eine Luftströmungskanal in Fluidverbindung mit dem Sensor (103) steht, wobei der mindestens eine Luftströmungskanal (105) angepasst ist, um ein elektromagnetisches Signal basierend auf einem Satz von geometrischen Parametern, die mit dem mindestens einen Luftströmungskanal (105) verbunden sind, zu blockieren; und **dadurch gekennzeichnet, dass**
der Hohlkörper (102) einen Verbindungsabschnitt (102a) umfasst, der angepasst ist, um den Hohlkörper (102) elektrisch mit einem Gehäuse (104) des Gasdetektors (101) zu verbinden und den Hohlkörper (102) entfernbar an einem Gehäuse (104) des Gasdetektors (101) zu befestigen.

2. Elektromagnetisches Abschirmelement (100) nach Anspruch 1, wobei der Satz von geometrischen Parametern mindestens eines von einer Form, einem Innendurchmesser, einer Ausrichtung und einer Länge des mindestens einen Luftströmungskanals umfasst.

3. Elektromagnetisches Abschirmelement (100) nach Anspruch 1 oder 2, wobei der Innendurchmesser des mindestens einen Luftströmungskanals (105) weniger als 5 mm beträgt.

4. Elektromagnetisches Abschirmelement (100) nach Anspruch 1, 2 oder 3, wobei der mindestens eine Luftströmungskanal (105) ausgerichtet ist, um zu verhindern, dass mindestens eines von Umgebungsfeinstaub und Wasserpartikeln direkt mit dem Sensor (103) in Kontakt kommt.

5. Elektromagnetisches Abschirmelement (100) nach einem vorstehenden Anspruch, wobei der mindestens eine Luftströmungskanal (105) in einer Neigung von mehr als 45 Grad relativ zu einer Längsachse (X-X') des Hohlkörpers (102) ausgerichtet ist.

6. Elektromagnetisches Abschirmelement (100) nach einem vorstehenden Anspruch, wobei der Verbindungsabschnitt (102a) auf einer Außenfläche (106a) einer Seitenwand (106) des Hohlkörpers (102) definiert ist.

7. Elektromagnetisches Abschirmelement (100) nach Anspruch 6, wobei die Seitenwand (106) mindestens einen Prallflächenabschnitt (106b) umfasst, der sich von der Seitenwand (106) in einer Richtung parallel zur Längsachse (X-X') des Hohlkörpers (102) nach unten erstreckt.

8. Elektromagnetisches Abschirmelement (100) nach einem vorstehenden Anspruch, wobei der Hohlkörper (102) mindestens einen Prallflächenabschnitt (106b) umfasst, der entfernbar an der Seitenwand (106) befestigt ist und sich in einer Richtung parallel zur Längsachse (X-X') des Hohlkörpers (102) nach unten erstreckt.

9. Elektromagnetisches Abschirmelement (100) nach einem vorstehenden Anspruch, wobei der mindestens eine Prallflächenabschnitt (106b) vom Hohlkörper (102) beabstandet ist, um das Eintreten von Umgebungsluft in den mindestens einen Luftströmungskanal (105) zu ermöglichen.

10. Elektromagnetisches Abschirmelement (100) nach Anspruch 9, wobei der mindestens eine Prallflächenabschnitt (106b) und der mindestens eine Luftströmungskanal (105) angeglichen sind, um das Eintreten von Umgebungsluft in den mindestens einen Luftströmungskanal (105) zu ermöglichen.

11. Gasdetektor (101), umfassend:
ein Gehäuse (104), das angepasst ist, um mindestens einen Abschnitt eines Sensors (103) und eine Steuerschaltung (107), die elektrisch mit dem Sensor (103) verbunden ist, zu umschließen; und
das elektromagnetische Abschirmelement (100) nach einem vorstehenden Anspruch, das angepasst ist, um entfernbar an dem Gehäuse (104) befestigt zu werden.

12. Gasdetektor (101) nach Anspruch 11, wenn abhängig von Anspruch 7, wobei der mindestens eine Prallflächenabschnitt (106b') angepasst ist, um ein sekundäres Abdeckelement (106c') entfernbar an der Seitenwand (106') zu befestigen.

## Revendications

1. Organe (100) de blindage électromagnétique pour un détecteur (101) de gaz, l'organe (100) de blindage électromagnétique comprenant :
un corps (102) creux adapté pour enfermer au moins partiellement un capteur (103), le corps creux comprenant :
au moins un canal (105) de flux d'air défini dans le corps (102) creux, l'au moins un canal de flux d'air étant en communication fluidique avec le capteur (103), dans lequel l'au moins un canal (105) de flux d'air est adapté pour bloquer un signal électromagnétique sur la base d'un ensemble de paramètres géométriques associés à l'au moins un canal (105) de flux d'air ; et **caractérisé en ce que**
le corps (102) creux comprend une partie (102a) connexion adaptée pour connecter électriquement le corps (102) creux à une enceinte (104) du détecteur (101) de gaz et pour fixer de manière amovible le corps (102) creux à une enceinte (104) du détecteur (101) de gaz.

2. Organe (100) de blindage électromagnétique selon la revendication 1, dans lequel l'ensemble de paramètres géométriques comprend au moins l'un parmi une forme, un diamètre interne, une orientation et une longueur de l'au moins un canal de flux d'air.

3. Organe (100) de blindage électromagnétique selon la revendication 1 ou la revendication 2, dans lequel le diamètre interne de l'au moins un canal (105) de flux d'air est inférieur à 5 mm.

4. Organe (100) de blindage électromagnétique selon la revendication 1, la revendication 2 ou la revendication 3, dans lequel l'au moins un canal (105) de flux d'air est orienté de manière à empêcher au moins l'une parmi une matière particulaire ambiante et des particules d'eau d'entrer directement en contact avec le capteur (103).

5. Organe (100) de blindage électromagnétique selon une quelconque revendication précédente, dans lequel l'au moins un canal (105) de flux d'air est orienté à une inclinaison supérieure à 45 degrés par rapport à un axe (X-X') longitudinal du corps (102) creux.

6. Organe (100) de blindage électromagnétique selon une quelconque revendication précédente, dans lequel la partie (102a) connexion est définie sur une surface (106a) externe d'une paroi (106) latérale du corps (102) creux.

7. Organe (100) de blindage électromagnétique selon la revendication 6, dans lequel la paroi (106) latérale comprend au moins une partie (106b) déflecteur s'étendant vers le bas à partir de la paroi (106) latérale dans une direction parallèle à l'axe (X-X') longitudinal du corps (102) creux.

8. Organe (100) de blindage électromagnétique selon une quelconque revendication précédente, dans lequel le corps (102) creux comprend au moins une partie (106b) déflecteur fixée de manière amovible à la paroi (106) latérale et s'étendant vers le bas dans une direction parallèle à l'axe (X-X') longitudinal du corps (102) creux.

9. Organe (100) de blindage électromagnétique selon une quelconque revendication précédente, dans lequel l'au moins une partie (106b) déflecteur est espacée du corps (102) creux pour permettre l'entrée de l'air ambiant dans l'au moins un canal (105) de flux d'air.

10. Organe (100) de blindage électromagnétique selon la revendication 9, dans lequel l'au moins une partie (106b) déflecteur et l'au moins un canal (105) de flux d'air sont alignés pour permettre l'entrée de l'air ambiant dans l'au moins un canal (105) de flux d'air.

11. Détecteur (101) de gaz comprenant :
une enceinte (104) adaptée pour enfermer au moins une partie d'un capteur (103) et une circuiterie (107) de commande connectée électriquement au capteur (103) ; et
l'organe (100) de blindage électromagnétique selon une quelconque revendication précédente adapté pour être fixé de manière amovible à l'enceinte (104).

12. Détecteur (101) de gaz selon la revendication 11 lorsqu'elle dépend de la revendication 7, dans lequel l'au moins une partie (106b') déflecteur est adaptée pour fixer de manière amovible un élément (106c') de couvercle secondaire à la paroi (106') latérale.
